(19) Europäisches Patentamt

European Patent Office

Office européen des brevets

(11) **EP 4 670 722 A1**

(12) **EUROPEAN PATENT APPLICATION**
published in accordance with Art. 153(4) EPC

(43) Date of publication:
**31.12.2025 Bulletin 2026/01**

(21) Application number: 24763723.4

(22) Date of filing: **20.02.2024**

(51) International Patent Classification (IPC):
*A61K 31/702* (2006.01)    *A61K 8/60* (2006.01)
*A61P 17/00* (2006.01)    *A61P 17/02* (2006.01)
*A61P 43/00* (2006.01)    *A61Q 1/00* (2006.01)
*A61Q 19/00* (2006.01)    *A61Q 19/08* (2006.01)

(52) Cooperative Patent Classification (CPC):
**A61K 8/60; A61K 31/702; A61P 17/00;
A61P 17/02; A61P 43/00; A61Q 1/00; A61Q 19/00;
A61Q 19/08**

(86) International application number:
**PCT/JP2024/006093**

(87) International publication number:
**WO 2024/181242 (06.09.2024 Gazette 2024/36)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **27.02.2023   JP 2023028616**

(71) Applicant: **Kyowa Pharma Chemical Co., Ltd.
Takaoka-shi, Toyama 933-8511 (JP)**

(72) Inventor: **SUGITA Ai
Takaoka-shi, Toyama 933-8511 (JP)**

(74) Representative: **Vossius & Partner
Patentanwälte Rechtsanwälte mbB
Siebertstrasse 3
81675 München (DE)**

(54) **PROPHYLACTIC OR AMELIORATING AGENT FOR CELL AGING**

(57)   A prophylactic or ameliorating agent for cell aging, comprising 2'-fucosyllactose.

**EP 4 670 722 A1**

## Description

### Technical Field

The present invention relates to a prophylactic or ameliorating agent for cell aging.

### Background Art

Human Milk Oligosaccharide (HMO) is an oligosaccharide contained in human breast milk. HMO is a substance that serves as a nutrient for beneficial bacteria such as bifidobacteria, and its various physiological functions are expected to be applied.

For example, Patent Literature 1 describes a growth inhibitor for pathogenic bacteria in the oral cavity, comprising one or more selected from milk oligosaccharide and lactulose. As HMOs, sialyllactose (Patent Literatures 2 to 11) and fucosyllactose (Patent Literatures 12 to 17) are known.

Incidentally, in recent years, with the progression of societal aging, technologies related to anti-aging have been attracting particular attention. The GLS1 gene, which is an anti-aging-related gene, is a gene related to glutamine metabolism and is known to be necessary for the survival of senescent cells. Therefore, for example, suppressing the expression of the GLS1 gene is sought in the prevention or amelioration of cell aging. For example, Patent Literature 18 describes a GLS1 gene inhibitor characterized by comprising a Euglena-derived substance as an active ingredient and being used to inhibit the expression of the GLS1 gene. Furthermore, it is also expected that cell aging can be prevented or ameliorated by an agent for ameliorating endoplasmic reticulum stress (for example, Patent Literature 19).

### Citation List

### Patent Literature

[Patent Literature 1] JP 2021-065176 A
[Patent Literature 2] KR 10-2019-0067499
[Patent Literature 3] WO 2012/053849
[Patent Literature 4] WO 2014/138578
[Patent Literature 5] WO 2000/006115
[Patent Literature 6] KR 10-2011-0092602
[Patent Literature 7] KR 10-2016-0009738
[Patent Literature 8] JP 2001-206848 A
[Patent Literature 9] JP H03-049648 A
[Patent Literature 10] JP H02-207089 A
[Patent Literature 11] WO 2016/172183
[Patent Literature 12] KR 10-2018-0051688
[Patent Literature 13] KR 10-2019-0045079
[Patent Literature 14] CN 109043542
[Patent Literature 15] EP 3127543
[Patent Literature 16] US 5,002,759
[Patent Literature 17] US 5,401,723
[Patent Literature 18] JP 2022-112464 A
[Patent Literature 19] JP 2020-011930 A

### Summary of Invention

### Technical Problem

An object of the present invention is to provide a novel prophylactic or ameliorating agent for cell aging.

### Solution to Problem

The present invention relates to, for example, the following [1] to [51].

[1] A prophylactic or ameliorating agent for cell aging, comprising 2'-fucosyllactose.

[2] The prophylactic or ameliorating agent according to [1], for promoting proliferation of normal cells.

[3] The prophylactic or ameliorating agent according to [1], for removing senescent cells.

[4] The prophylactic or ameliorating agent according to any one of [1] to [3], wherein the cell aging is cell aging caused by GLS1 expression.

[5] The prophylactic or ameliorating agent according to any one of [1] to [3], wherein the cell aging is cell aging caused by endoplasmic reticulum stress.

[6] The prophylactic or ameliorating agent according to any one of [1] to [5], wherein the cell is a skin cell.

[7] The prophylactic or ameliorating agent according to any one of [1] to [6], wherein the cell is an epidermal keratinocyte.

[8] The prophylactic or ameliorating agent according to any one of [1] to [6], wherein the cell is a dermal fibroblast.

[9] The prophylactic or ameliorating agent according to any one of [1] to [8], which is at least one selected from the group consisting of an agent for treating skin wounds, an agent for promoting skin metabolism, a prophylactic or ameliorating agent for blemishes, a prophylactic or ameliorating agent for wrinkles, a prophylactic or ameliorating agent for sagging, and a prophylactic or ameliorating agent for rough skin.

[9a] The prophylactic or ameliorating agent according to any one of [1] to [5], wherein the cell is a kidney cell, a stomach cell, a microglial cell, a gingival cell, or a vascular endothelial cell.

[9b] The prophylactic or ameliorating agent according to any one of [1] to [5], and [9a], which is at least one selected from the group consisting of a prophylactic or ameliorating agent for glomerular sclerosis, a prophylactic or ameliorating agent for decline in renal function, a prophylactic or ameliorating agent for decrease in gastric acid secretion from stomach parietal cells, a prophylactic or ameliorating agent for decrease in pepsinogen secretion from stomach chief cells, a prophylactic or ameliorating agent for cognitive decline, a prophylactic or ameliorating agent for decrease in saliva secretion, a prophylactic or ameliorating agent for inflammation in gingiva, a prophylactic or ameliorating agent for arteriosclerosis, and a prophylactic or ameliorating agent for obesity, diabetes, or impaired glucose tolerance.

[10] The prophylactic or ameliorating agent according to any one of [1] to [9b], which is a topical agent.

[11] The prophylactic or ameliorating agent according to any one of [1] to [10], which is a cosmetic.

[11a] The prophylactic or ameliorating agent according to any one of [1] to [9b], which is a food product.

[12] A method for preventing or ameliorating cell aging, comprising administering 2'-fucosyllactose to a subject in need thereof.

[13] A method for promoting proliferation of normal cells, comprising administering 2'-fucosyllactose to a subject in need thereof.

[14] A method for removing senescent cells, comprising administering 2'-fucosyllactose to a subject in need thereof.

[15] The method according to [12], wherein the cell aging is cell aging caused by GLS1 expression.

[16] The method according to [12], wherein the cell aging is cell aging caused by endoplasmic reticulum stress.

[17] The method according to any one of [12] to [16], wherein the cell is a skin cell.

[18] The method according to any one of [12] to [17], wherein the cell is an epidermal keratinocyte.

[19] The method according to any one of [12] to [17], wherein the cell is a dermal fibroblast.

[20] A method for treating skin wounds, promoting skin metabolism, preventing or ameliorating blemishes, preventing or ameliorating wrinkles, preventing or ameliorating sagging, or preventing or ameliorating rough skin, comprising administering 2'-fucosyllactose to a subject in need thereof.

[20a] The method according to any one of [12] to [16], wherein the cell is a kidney cell, a stomach cell, a microglial cell, a gingival cell, or a vascular endothelial cell.

[20b] A method for preventing or ameliorating glomerular sclerosis, preventing or ameliorating decline in renal function, preventing or ameliorating decrease in gastric acid secretion from stomach parietal cells, preventing or ameliorating decrease in pepsinogen secretion from stomach chief cells, preventing or ameliorating cognitive decline, preventing or ameliorating decrease in saliva secretion, preventing or ameliorating inflammation in gingiva, preventing or ameliorating arteriosclerosis, or preventing or ameliorating obesity, diabetes, or impaired glucose tolerance, comprising administering 2'-fucosyllactose to a subject in need thereof.

[21] The method according to any one of [12] to [20b], wherein the 2'-fucosyllactose is transdermally administered.

[21a] The method according to any one of [12] to [20b], wherein the 2'-fucosyllactose is orally ingested.

[22] 2'-Fucosyllactose for use in preventing or ameliorating cell aging for therapeutic use.

[23] 2'-Fucosyllactose for use in promoting proliferation of normal cells for therapeutic use.

[24] 2'-Fucosyllactose for use in removing senescent cells for therapeutic use.

[25] 2'-Fucosyllactose for use according to [22], wherein the cell aging is cell aging caused by GLS1 expression.

[26] 2'-Fucosyllactose for use according to [22], wherein the cell aging is cell aging caused by endoplasmic reticulum stress.

[27] 2'-Fucosyllactose for use according to any one of [22] to [26], wherein the cell is a skin cell.

EP 4 670 722 A1

[28] 2'-Fucosyllactose for use according to any one of [22] to [27], wherein the cell is an epidermal keratinocyte.

[29] 2'-Fucosyllactose for use according to any one of [22] to [27], wherein the cell is a dermal fibroblast.

[30] 2'-Fucosyllactose for use in at least one selected from the group consisting of treating skin wounds and promoting skin metabolism for therapeutic use.

[30a] 2'-Fucosyllactose for use according to any one of [22] to [26], wherein the cell is a kidney cell, a stomach cell, a microglial cell, a gingival cell, or a vascular endothelial cell.

[30b] 2'-Fucosyllactose for use in at least one selected from the group consisting of preventing or ameliorating glomerular sclerosis, preventing or ameliorating decline in renal function, preventing or ameliorating decrease in gastric acid secretion from stomach parietal cells, preventing or ameliorating decrease in pepsinogen secretion from stomach chief cells, preventing or ameliorating cognitive decline, preventing or ameliorating decrease in saliva secretion, preventing or ameliorating inflammation in gingiva, preventing or ameliorating arteriosclerosis, and preventing or ameliorating obesity, diabetes, or impaired glucose tolerance, for therapeutic use.

[31] 2'-Fucosyllactose for use according to any one of [22] to [30b], for transdermal use.

[31a] 2'-Fucosyllactose for use according to any one of [22] to [30b], for oral use.

[32] Use of 2'-fucosyllactose for preventing or ameliorating cell aging for non-therapeutic use.

[33] Use of 2'-fucosyllactose for promoting proliferation of normal cells for non-therapeutic use.

[34] Use of 2'-fucosyllactose for removing senescent cells for non-therapeutic use.

[35] The use according to [32], wherein the cell aging is cell aging caused by GLS 1 expression.

[36] The use according to [32], wherein the cell aging is cell aging caused by endoplasmic reticulum stress.

[37] The use according to any one of [32] to [36], wherein the cell is a skin cell.

[38] The use according to any one of [32] to [37], wherein the cell is an epidermal keratinocyte.

[39] The use according to any one of [32] to [37], wherein the cell is a dermal fibroblast.

[40] Use of 2'-fucosyllactose for at least one selected from the group consisting of preventing or ameliorating blemishes, preventing or ameliorating wrinkles, preventing or ameliorating sagging, and preventing or ameliorating rough skin, for non-therapeutic use.

[40a] The use according to any one of [32] to [36], wherein the cell is a kidney cell, a stomach cell, a microglial cell, a gingival cell, or a vascular endothelial cell.

[40b] Use of 2'-fucosyllactose for at least one selected from the group consisting of preventing or ameliorating glomerular sclerosis, preventing or ameliorating decline in renal function, preventing or ameliorating decrease in gastric acid secretion from stomach parietal cells, preventing or ameliorating decrease in pepsinogen secretion from stomach chief cells, preventing or ameliorating cognitive decline, preventing or ameliorating decrease in saliva secretion, preventing or ameliorating inflammation in gingiva, preventing or ameliorating arteriosclerosis, and preventing or ameliorating obesity, diabetes, or impaired glucose tolerance, for non-therapeutic use.

[41] The use according to any one of [32] to [40b], which is transdermal use.

[41a] The use according to any one of [32] to [40b], which is oral use.

[42] Use of 2'-fucosyllactose for the manufacture of a prophylactic or ameliorating agent for cell aging.

[43] Use of 2'-fucosyllactose for the manufacture of an agent for promoting proliferation of normal cells.

[44] Use of 2'-fucosyllactose for the manufacture of an agent for removing senescent skin cells.

[45] The use according to [42], wherein the cell aging is cell aging caused by GLS 1 expression.

[46] The use according to [42], wherein the cell aging is cell aging caused by endoplasmic reticulum stress.

[47] The use according to any one of [42] to [46], wherein the cell is a skin cell.

[48] The use according to any one of [42] to [47], wherein the cell is an epidermal keratinocyte.

[49] The use according to any one of [42] to [47], wherein the cell is a dermal fibroblast.

[50] Use of 2'-fucosyllactose for the manufacture of at least one agent selected from the group consisting of an agent for treating skin wounds, an agent for promoting skin metabolism, a prophylactic or ameliorating agent for blemishes, a prophylactic or ameliorating agent for wrinkles, a prophylactic or ameliorating agent for sagging, and a prophylactic or ameliorating agent for rough skin.

[50a] The use according to any one of [42] to [46], wherein the cell is a kidney cell, a stomach cell, a microglial cell, a gingival cell, or a vascular endothelial cell.

[50b] Use of 2'-fucosyllactose for the manufacture of at least one agent selected from the group consisting of a prophylactic or ameliorating agent for glomerular sclerosis, a prophylactic or ameliorating agent for decline in renal function, a prophylactic or ameliorating agent for decrease in gastric acid secretion from stomach parietal cells, a prophylactic or ameliorating agent for decrease in pepsinogen secretion from stomach chief cells, a prophylactic or ameliorating agent for cognitive decline, a prophylactic or ameliorating agent for decrease in saliva secretion, a prophylactic or ameliorating agent for inflammation in gingiva, a prophylactic or ameliorating agent for arteriosclerosis, and a prophylactic or ameliorating agent for obesity, diabetes, or impaired glucose tolerance.

[51] The use according to any one of [42] to [50b], wherein the agent is a topical agent.

[51a] The use according to any one of [42] to [50b], wherein the agent is an oral agent.

**Advantageous Effects of Invention**

According to the present invention, a novel prophylactic or ameliorating agent for cell aging can be provided.

**Brief Description of Drawings**

FIG. 1 is a graph showing the cell proliferation rate of normal epidermal keratinocytes.
FIG. 2 is a graph showing the cell proliferation rate of normal dermal fibroblasts.
FIG. 3 is a graph showing the cell proliferation rate of senescent dermal fibroblasts.
FIG. 4 is a graph showing the GLS1 expression suppressing effect of 2'-fucosyllactose (2'-FL).
FIG. 5 is a graph showing the cell viability of epidermal keratinocytes treated with endoplasmic reticulum stress.
FIG. 6 is a graph showing the cell viability of dermal fibroblasts treated with endoplasmic reticulum stress.
FIG. 7 is a graph showing the laminin secretion restoring effect of 2'-FL in epidermal keratinocytes.
FIG. 8 is a graph showing the laminin secretion restoring effect of 2'-FL in dermal fibroblasts.
FIG. 9 is a graph showing the type I collagen expression promoting effect of 2'-FL in dermal fibroblasts.
FIG. 10 is a graph showing the IL-1$\alpha$ expression suppressing effect of 2'-FL in dermal fibroblasts.
FIG. 11 is a graph showing the results of verifying the GLS1 expression suppressing effect of lactose in kidney cells, stomach cells, microglial cells, gingival cells, or vascular endothelial cells.
FIG. 12 is a graph showing the GLS1 expression suppressing effect of 2'-FL in kidney cells, stomach cells, microglial cells, gingival cells, or vascular endothelial cells.

**Description of Embodiments**

A prophylactic or ameliorating agent for cell aging according to one embodiment comprises 2'-fucosyllactose (2'-FL). 2'-FL is a trisaccharide composed of L-fucose, D-galactose, and D-glucose, and is a compound in which the 2'-position of lactose is fucosylated. As 2'-FL, one purified after being synthesized by Escherichia coli may be used, or a commercially available product may be used.

The prophylactic or ameliorating agent for cell aging according to one embodiment can inhibit GLS1 gene expression by comprising 2'-FL. That is, the prophylactic or ameliorating agent for cell aging according to one embodiment may be a GLS1 expression inhibitor or a GLS1 gene expression inhibitor. To "inhibit GLS1 gene expression" includes not only inhibiting expression at the transcriptional level (expression as mRNA) but also inhibiting expression at the translational level (expression as protein).

When GLS1 gene expression is inhibited, senescent cells are removed. Therefore, the GLS1 expression inhibitor according to one embodiment can be used for removing senescent cells, and may be an agent for removing senescent cells, comprising 2'-FL. In the present specification, "agent for removing senescent cells" means one used for removing senescent cells in skin tissue. "Removing senescent cells" means removing senescent cells from a tissue or killing senescent cells in a tissue. "Removing senescent cells" means, for example, inducing cell death in senescent cells in vivo or in vitro, or selectively removing senescent cells from a cell population containing senescent cells. Since the GLS1 expression inhibitor and the agent for removing senescent cells according to one embodiment have an action of inhibiting GLS1 gene expression, it is possible to selectively kill senescent cells. Therefore, according to the GLS1 expression inhibitor and the agent for removing senescent cells according to one embodiment, accumulation of senescent cells in tissues (e.g., skin tissue, kidney, stomach, brain, spinal cord, gingiva, blood vessel, etc.) can be suppressed, and a reduction in the health of tissues (e.g., skin, kidney, stomach, brain, spinal cord, gingiva, blood vessel, etc.) can be suppressed.

The prophylactic or ameliorating agent for cell aging according to one embodiment can promote proliferation of normal cells by comprising 2'-FL. That is, the prophylactic or ameliorating agent for cell aging according to one embodiment may be an agent for promoting proliferation of normal cells, comprising 2'-FL. Since the prophylactic or ameliorating agent for cell aging according to one embodiment can promote proliferation of normal cells also at a wound site of a tissue (e.g., skin, kidney, stomach, brain, spinal cord, gingiva, blood vessel, etc.), it can promote wound healing. Therefore, the prophylactic or ameliorating agent for cell aging according to one embodiment can be used for the purpose of treating tissue wounds, and may be an agent for treating tissue wounds. The prophylactic or ameliorating agent for cell aging according to one embodiment can be used for the purpose of treating skin wounds, and may be an agent for treating skin wounds.

The prophylactic or ameliorating agent for cell aging according to one embodiment, by comprising 2'-FL, has an action of promoting proliferation of normal cells and removing senescent cells. Therefore, the prophylactic or ameliorating agent for cell aging according to one embodiment can be used for promoting proliferation of normal cells and removing senescent cells.

The prophylactic or ameliorating agent for cell aging according to one embodiment can be used for at least one purpose selected from the group consisting of treating tissue wounds and promoting cell metabolism. That is, the prophylactic or ameliorating agent for cell aging according to one embodiment may be at least one selected from the group consisting of an agent for treating tissue wounds and an agent for promoting cell metabolism, comprising 2'-FL. The prophylactic or ameliorating agent for cell aging according to one embodiment can be used for at least one purpose selected from the group consisting of treating skin wounds, promoting skin metabolism, preventing or ameliorating blemishes, preventing or ameliorating wrinkles, preventing or ameliorating sagging, and preventing or ameliorating rough skin. That is, the prophylactic or ameliorating agent for cell aging according to one embodiment may be at least one selected from the group consisting of an agent for treating skin wounds, an agent for promoting skin metabolism, a prophylactic or ameliorating agent for blemishes, a prophylactic or ameliorating agent for wrinkles, a prophylactic or ameliorating agent for sagging, and a prophylactic or ameliorating agent for rough skin, comprising 2'-FL.

The prophylactic or ameliorating agent for cell aging according to one embodiment, by comprising 2'-FL, can promote type I collagen expression in cells, can more effectively promote type I collagen expression in skin cells, and can further effectively promote type I collagen expression in dermal fibroblasts. Type I collagen present in the skin, particularly in the dermis, can impart elasticity and strength to the skin. Therefore, the prophylactic or ameliorating agent for cell aging according to one embodiment can be used as a prophylactic or ameliorating agent for wrinkles and/or a prophylactic or ameliorating agent for sagging, also because it can increase type I collagen in the skin, particularly in the dermis.

The prophylactic or ameliorating agent for cell aging according to one embodiment, by comprising 2'-FL, can suppress IL-1$\alpha$ expression due to an inflammatory response in cells, can more effectively suppress IL-1$\alpha$ expression in skin cells, and can further effectively suppress IL-1$\alpha$ expression in dermal fibroblasts. Suppression of IL-1$\alpha$ due to an inflammatory response in the epidermis or dermis can exert effects such as keeping the skin whitened, suppressing sunburn, and ameliorating atopic symptoms. Therefore, the prophylactic or ameliorating agent for cell aging according to one embodiment can be used as a prophylactic or ameliorating agent for blemishes and/or a prophylactic or ameliorating agent for rough skin, also because it can suppress IL-1$\alpha$ expression due to an inflammatory response in skin cells. The inflammatory response may be an inflammatory response induced by reactive oxygen species (ROS) such as hydrogen peroxide ($H_2O_2$).

Cells targeted by the prophylactic or ameliorating agent for cell aging according to one embodiment may be animal cells, and may be, for example, cells of tissues or organs such as skin, muscle, bone, joint, adipose tissue, brain, spinal cord, digestive organ, reproductive organ, endocrine organ, respiratory organ, circulatory system, immune system, bone, joint, and adipose tissue. In any of these tissues and organs, GLS1 can be expressed, and endoplasmic reticulum stress can also occur. Cells targeted by the prophylactic or ameliorating agent for cell aging according to one embodiment may be skin cells. Examples of skin cells include cells constituting the epidermis and dermis. Examples of cells constituting the epidermis include epidermal keratinocytes (keratinocytes), pigment cells (melanocytes), Langerhans cells, and Merkel cells, and among these, epidermal keratinocytes are preferable. The cells constituting the epidermis are preferably cells present in the basal layer, spinous layer, granular layer, or stratum corneum of the epidermis. Examples of cells constituting the dermis include fibroblasts, macrophages, mast cells, plasma cells, and dermal dendritic cells, and among these, dermal fibroblasts are preferable. The cells constituting the dermis are preferably cells present in the papillary layer or reticular layer of the dermis.

It is also preferable that cells targeted by the prophylactic or ameliorating agent for cell aging according to one embodiment are kidney cells. Examples of kidney cells include glomerular cells, tubular cells, interstitial cells, and the like. In kidney cells, suppressing GLS1 gene expression can prevent or ameliorate glomerular sclerosis, or prevent or ameliorate decline in renal function. The above-mentioned glomerular sclerosis and decline in renal function may be due to aging of kidney cells. For example, Johmura, Yoshikazu, et al. "Senolysis by glutaminolysis inhibition ameliorates various age-associated disorders." Science 371.6526 (2021): 265-270. describes that when GLS1 was inhibited in 80-week-old male mice, age-associated glomerular sclerosis, macrophage infiltration into the kidney, and renal dysfunction were ameliorated. Therefore, the prophylactic or ameliorating agent for cell aging according to one embodiment can be used as a prophylactic or ameliorating agent for glomerular sclerosis, or a prophylactic or ameliorating agent for decline in renal function.

It is also preferable that cells targeted by the prophylactic or ameliorating agent for cell aging according to one embodiment are stomach cells. Examples of stomach cells include chief cells, mucous neck cells, stomach parietal cells, and the like. In chief cells, suppressing GLS1 gene expression can prevent or ameliorate a decrease in pepsinogen secretion from the chief cells. The decrease in pepsinogen secretion may be due to aging of chief cells. For example, https://www.tyojyu.or.jp/net/kenkou-tyoju/rouka/saibou-kotai-rouka.html (accessed February 6, 2024) describes that when the stomach ages, pepsinogen secretion from chief cells decreases. Therefore, the prophylactic or ameliorating agent for cell aging according to one embodiment can be used as a prophylactic or ameliorating agent for decline in renal function.

Furthermore, in stomach parietal cells, suppressing GLS1 gene expression can prevent or ameliorate a decrease in gastric acid secretion from the stomach parietal cells. The decrease in gastric acid secretion may be due to aging of stomach parietal cells. For example, https://www.tyojyu.or.jp/net/kenkou-tyoju/rouka/saibou-kotai-rouka.html (accessed February 6, 2024) describes that when the stomach ages, gastric acid secretion from stomach parietal cells decreases. Therefore, the prophylactic or ameliorating agent for cell aging according to one embodiment can be used as a prophylactic or ameliorating agent for decrease in gastric acid secretion.

It is also preferable that cells targeted by the prophylactic or ameliorating agent for cell aging according to one embodiment are microglial cells. In microglial cells, suppressing GLS1 gene expression can prevent or ameliorate cognitive decline. The cognitive decline may be due to aging of microglial cells. For example, Shohei Kasai, "Removal of senescent glial cells: a key to preventing cognitive decline?" Pharmacia 55.9 (2019): 889-889, describes that in mice from which senescent microglial cells were removed, the amount of phosphorylated tau protein in the brain was significantly reduced, brain atrophy was alleviated, reduction in the area of the hippocampal dentate gyrus was suppressed, and cognitive function was improved. Therefore, the prophylactic or ameliorating agent for cell aging according to one embodiment can be used as a prophylactic or ameliorating agent for cognitive decline.

It is also preferable that cells targeted by the prophylactic or ameliorating agent for cell aging according to one embodiment are gingival cells. In gingival cells, suppressing GLS1 gene expression can prevent or ameliorate inflammation in the gingiva. The inflammation in the gingiva may be due to aging of gingival cells. For example, https://www.ncgg.go.jp/ncgg-kenkyu/documents/2021/21xx_6.pdf (accessed February 6, 2024) describes that in the gingiva of aged mice, a significant increase in the expression of IL-1$\beta$ and TNF$\alpha$ was observed compared to young mice. Therefore, the prophylactic or ameliorating agent for cell aging according to one embodiment can be used as a prophylactic or ameliorating agent for inflammation in gingiva.

It is also preferable that cells targeted by the prophylactic or ameliorating agent for cell aging according to one embodiment are vascular endothelial cells. In vascular endothelial cells, suppressing GLS1 gene expression can prevent or ameliorate arteriosclerosis. The arteriosclerosis may be due to aging of vascular endothelial cells. For example, Johmura, Yoshikazu, et al. "Senolysis by glutaminolysis inhibition ameliorates various age-associated disorders." Science 371.6526 (2021): 265-270. describes that when GLS1 was inhibited in mice in which arteriosclerosis of the thoracic aorta was induced, the arteriosclerosis was alleviated. Therefore, the prophylactic or ameliorating agent for cell aging according to one embodiment can be used as a prophylactic or ameliorating agent for arteriosclerosis.

Furthermore, in vascular endothelial cells, suppressing GLS1 gene expression can prevent or ameliorate obesity, diabetes, or impaired glucose tolerance. The obesity, diabetes, or impaired glucose tolerance may be due to aging of vascular endothelial cells. For example, Masayoshi Suda, Ippei Shimizu, and Toru Minamino, "On vascular endothelial cell senescence." Japanese Journal of Thrombosis and Hemostasis 30.3 (2019): 521-528, describes that diabetes promotes aging of vascular cells; vascular endothelium-specific p53-deficient mice, in which aging of vascular endothelium was suppressed, showed suppressed obesity compared to wild type even when fed a high-fat diet, and systemic impaired glucose tolerance was mild; on the other hand, vascular endothelial cell senescence-accelerated mice, in which Mdm2/Mdm4 that degrades p53 was specifically deleted in vascular endothelial cells causing p53 overexpression in vascular endothelial cells, showed exacerbated impaired glucose tolerance upon high-fat diet loading, and accelerated obesity. Therefore, the prophylactic or ameliorating agent for cell aging according to one embodiment can be used as a prophylactic or ameliorating agent for obesity, diabetes, or impaired glucose tolerance.

In the present invention, "normal cell" means a cell that can continue cell proliferation and cell cycle. If the expression level of a senescence marker in a certain cell is comparable to the normal expression level of the senescence marker in cells capable of continuing proliferation, that cell can be regarded as a normal cell. Examples of senescence markers include acid $\beta$-galactosidase (SA-$\beta$-gal), p53, P16INK4a, P21CIP1, and the like.

In the present invention, "senescent cell" means a cell in which cell proliferation or cell cycle has irreversibly stopped. If the expression level of a senescence marker in a certain cell is significantly increased compared to the normal expression level of the senescence marker in normal cells, that cell can be regarded as a senescent cell.

The prophylactic or ameliorating agent for cell aging according to one embodiment, by comprising 2'-FL, can ameliorate endoplasmic reticulum stress in cells. That is, the prophylactic or ameliorating agent for cell aging according to one embodiment may be an agent for ameliorating endoplasmic reticulum stress in cells. Endoplasmic reticulum stress refers to a state where proteins (denatured proteins) that have not been folded into a normal higher-order structure due to gene mutation, viral infection, inflammation, harmful chemical substances, or the like, accumulate in the endoplasmic reticulum, thereby causing adverse effects on cells. Cells in which endoplasmic reticulum stress has occurred tend to lose normal functions, and apoptosis may also be induced. It is assumed that due to functional decline of cells, apoptosis, or the like, metabolism may decrease, or blemishes, wrinkles, sagging, rough skin, or the like may occur. It is considered that ameliorating endoplasmic reticulum stress in cells leads to preventing or ameliorating aging of the tissue to which the cells belong. Furthermore, since the prophylactic or ameliorating agent for cell aging according to one embodiment can also ameliorate endoplasmic reticulum stress caused by inflammation occurring at a tissue wound or the like, it can also be used for the purpose of treating tissue wounds.

When endoplasmic reticulum stress is induced in cells, the laminin secretion amount from the cells decreases. The prophylactic or ameliorating agent for cell aging according to one embodiment, by comprising 2'-FL, can restore the decrease in laminin secretion amount due to endoplasmic reticulum stress in cells, can more effectively restore the decrease in laminin secretion amount due to endoplasmic reticulum stress in skin cells, and can further effectively restore the decrease in laminin secretion amount due to endoplasmic reticulum stress in epidermal keratinocytes and dermal fibroblasts. Therefore, the prophylactic or ameliorating agent for cell aging according to one embodiment can be used as an agent for ameliorating endoplasmic reticulum stress in cells, also because it can restore the decrease in laminin secretion amount even when endoplasmic reticulum stress is induced in cells.

Furthermore, laminin is one of the proteins constituting the basement membrane, which is present, for example, between the epidermis and the dermis, and can promote cell adhesion, cell migration, cell proliferation, and the like of cells such as skin cells. Therefore, also because it can restore the decrease in laminin secretion amount and promote cell adhesion, cell migration, cell proliferation, and the like of skin cells even when endoplasmic reticulum stress is induced in skin cells, it can be used for at least one purpose selected from the group consisting of treating skin wounds, promoting skin metabolism, preventing or ameliorating blemishes, preventing or ameliorating wrinkles, preventing or ameliorating sagging, and preventing or ameliorating rough skin.

Subjects to which the prophylactic or ameliorating agent for cell aging according to one embodiment can be applied include humans and non-human mammals, with humans being preferable. Examples of non-human mammals include mice, rats, guinea pigs, hamsters, rabbits, cats, dogs, sheep, pigs, cows, horses, goats, and monkeys.

When the prophylactic or ameliorating agent for cell aging according to one embodiment is applied to the skin, examples of sites of application include any body surface such as the scalp, face (forehead, cheeks, lips, nose, ears, etc.), neck, shoulders, back, chest, abdomen, genitals, arms, hands, lower limbs, feet, nails, hair, and the like. The prophylactic or ameliorating agent for cell aging according to one embodiment can be applied to the liver, kidney, spleen, large intestine, small intestine, stomach, bladder, and the like.

The prophylactic or ameliorating agent for cell aging according to one embodiment preferably comprises 2'-FL in an amount of 0.001% by mass or more and 5% by mass or less, and more preferably 0.01% by mass or more and 0.5% by mass or less, based on the total amount of the agent.

The dosage of the prophylactic or ameliorating agent for cell aging according to one embodiment can be, for a subject to be administered weighing 60 kg, 1 mg or more and 60 g or less, 0.01 mg or more and 1 g or less, 6 mg or more and 30 g or less, or 60 mg or more and 15 g or less per day.

The number of administrations of the prophylactic or ameliorating agent for cell aging according to one embodiment can be once a day or twice a day.

The administration method of the prophylactic or ameliorating agent for cell aging according to one embodiment may be transdermal administration, oral administration, intravenous administration, subcutaneous administration, intramuscular administration, intraperitoneal administration, or ophthalmic administration, and transdermal administration is preferable. That is, the prophylactic or ameliorating agent for cell aging according to one embodiment may be a topical agent. Examples of transdermal administration methods include a method of applying to a body surface such as the skin or a method of affixing to the skin.

The prophylactic or ameliorating agent for cell aging according to one embodiment may have the form of, for example, a solid; a liquid (solution or suspension); an emulsion such as a milky lotion or cream; a paste; a gel; or a mousse.

The prophylactic or ameliorating agent for cell aging according to one embodiment can be applied to both therapeutic use and non-therapeutic use (e.g., use for cosmetic purpose). Specifically, for example, the prophylactic or ameliorating agent for cell aging according to one embodiment may be a pharmaceutical product, a quasi-drug, a cosmetic, or a food product, and is preferably a cosmetic. Pharmaceutical products, quasi-drugs, cosmetics, and food products comprising the prophylactic or ameliorating agent for cell aging according to one embodiment can each be manufactured according to conventional methods. The content of the prophylactic or ameliorating agent for cell aging according to one embodiment or 2'-FL in pharmaceutical products, quasi-drugs, cosmetics, or food products is not particularly limited and can be freely set according to the purpose. The prophylactic or ameliorating agent for cell aging according to one embodiment may be used, ingested, or administered prophylactically to promote proliferation of normal cells and/or remove senescent cells. When the prophylactic or ameliorating agent for cell aging according to one embodiment is a food product, the ingestion may be oral ingestion.

The prophylactic or ameliorating agent for cell aging according to one embodiment can comprise components other than 2'-FL to the extent that the effects as a prophylactic or ameliorating agent for cell aging are not impaired. Specific examples of components other than 2'-FL include, for example, moisturizing ingredients, anti-inflammatory ingredients, antibacterial ingredients, cell-activating ingredients, anti-aging ingredients, blood circulation promoting ingredients, UV protection ingredients, whitening ingredients, vitamins, proteins, peptides, amino acids, alcohols, and the like. These active ingredients can be used alone or in combination of two or more kinds, as necessary.

When the prophylactic or ameliorating agent for cell aging according to one embodiment is a pharmaceutical product, a quasi-drug, a cosmetic, or a food product, the pharmaceutical product, quasi-drug, cosmetic, or food product may comprise, in addition to the prophylactic or ameliorating agent for cell aging according to one embodiment, components that can be usually used in pharmaceutical products, quasi-drugs, cosmetics, or food products. The pharmaceutical product, quasi-drug, cosmetic, or food product according to one embodiment may comprise bases, carriers, additives, and the like that are usually used in pharmaceutical products, quasi-drugs, cosmetics, or food products. Examples of additives include excipients, oils, powders, buffering agents, solubilizing agents, antioxidants, surfactants, thickeners, preservatives, pH adjusters, chelating agents, stabilizers, irritation-reducing agents, antiseptics, pigments, coloring agents, fragrances, glossing agents, gelling agents, alcohols, water-soluble polymers, film-forming agents, resins, keratolytic agents, and the like. The bases, carriers, and various additives mentioned above can be used alone or in combination of two or more kinds, as necessary.

When the prophylactic or ameliorating agent for cell aging according to one embodiment is a pharmaceutical product, the dosage form of the pharmaceutical product may be, for example, an aerosol, a solution, a suspension, an emulsion, a cream, an ointment, a gel, a liniment, a lotion, a poultice, a tape, an eye drop, a nasal drop, an ear drop, a suppository, an elixir, a capsule, a granule, a pill, a powder, a tablet, a syrup, an injection, a troche, or the like. When the prophylactic or ameliorating agent for cell aging according to one embodiment is used as a pharmaceutical product for the skin, it is preferably a topical agent. By making it a topical agent, 2'-FL can directly act on skin cells, and can exert its effects more strongly. As a topical agent, it preferably has a dosage form such as an aerosol, a solution, a suspension, an emulsion, a cream, an ointment, a gel, a liniment, a lotion, a poultice, a tape, or the like.

When the prophylactic or ameliorating agent for cell aging according to one embodiment is a quasi-drug or a cosmetic, the form of the quasi-drug or cosmetic may be, for example, basic skin care products such as skin lotion, milky lotion, cream, gel, beauty essence, sunscreen cosmetic, pack, hand cream, foot cream, body lotion, body cream; cleansing cosmetics such as facial cleanser, makeup remover, soap, body shampoo, shampoo, rinse, conditioner, nail polish remover; makeup cosmetics such as foundation, makeup base, lip cream, lipstick, cheek color, eye color, eyebrow pencil, manicure, hair color; antiperspirants; bath additives; perfumes; food and drink products such as food with nutrient function claims, food with function claims, food for specified health uses (FOSHU), food for special dietary uses, and the like. When used as a quasi-drug or cosmetic for the skin, it is also preferably a topical agent, just as with pharmaceutical products. By making it a topical agent, for example, 2'-FL can directly act on skin cells, and can exert its effects more strongly. Quasi-drugs or cosmetics as topical agents are preferably quasi-drugs and cosmetics in forms excluding food and drink products.

When the prophylactic or ameliorating agent for cell aging according to one embodiment is a food product, the food product may be, in addition to general food products, food for specified health uses (FOSHU), food with nutrient function claims, food with function claims, food for hospital patients, supplements, and the like. Furthermore, the prophylactic or ameliorating agent for cell aging according to one embodiment can also be used as a food additive.

Examples of the food composition include seasonings, processed meat products, processed agricultural products, beverages (lactic acid bacteria beverages, soft drinks, alcoholic beverages, carbonated beverages, milk beverages, fruit juice beverages, tea, coffee, nutritional drinks, etc.), powdered beverages (powdered juice, powdered soup, etc.), concentrated beverages, confectionery (candy (throat lozenges), cookies, biscuits, gum, gummies, chewables, tablets, chocolate, etc.), bread, cereals, and the like. Furthermore, in the case of food for specified health uses (FOSHU), food with nutrient function claims, food with function claims, and the like, they may be in the form of capsules, troches, syrups, granules, powders, or the like.

Here, food for specified health uses (FOSHU) is a food product comprising health functional ingredients that affect physiological functions and the like, and for which it is possible to display that it is suitable for specific health uses with the permission of the Commissioner of the Consumer Affairs Agency. In the present invention, it may be a food product sold with a display of specific health uses related to GLS1 inhibition, amelioration of endoplasmic reticulum stress, removal of senescent cells, or promotion of proliferation of normal cells.

Furthermore, food with nutrient function claims is a food product used for supplementing nutritional components (vitamins, minerals), and displays the functions of the nutritional components. To be sold as a food with nutrient function claims, the amount of nutritional components contained in the recommended daily intake must be within the range of specified upper and lower limits, and it is necessary to display not only the nutrient function claims but also cautionary statements and the like.

Furthermore, food with function claims is a food product for which functionality based on scientific evidence is displayed under the responsibility of the business operator. Information regarding the basis of safety and functionality is submitted to the Commissioner of the Consumer Affairs Agency before sale.

**Examples**

Hereinafter, the present invention will be described in detail with reference to examples, but the present invention is not limited by these examples.

[Example 1]

The epidermal cell proliferation activity of 2'-fucosyllactose was evaluated.

[Example 1-1: Absorbance Measurement]

Human normal epidermal keratinocytes (HaCaT) were cultured using DMEM containing 10% FBS. The cultured cells were collected by trypsin treatment. The collected cells were seeded in a 96-well microplate at a concentration of 5.0 x $10^3$ cells/well and cultured overnight. Thereafter, the cells were cultured for 2 days at 37°C in a medium for epidermal keratinocyte proliferation (FBS-free) to which a test sample (sucrose or 2'-fucosyllactose) was added to a concentration of 50 μg/mL, 100 μg/mL, or 200 μg/mL. The medium was replaced with FBS-free DMEM medium, then 10 μL/well of 5 mg/mL 3-[4,5-dimethylthiazol-2-yl]-2,5-diphenyltetrazolium bromide (MTT) was added, and the cells were cultured at 37°C for 3 hours. After completion of culture, formazan produced in the cells was extracted with 100 μL of dimethyl sulfoxide (DMSO). After extraction, the absorbance of formazan (As, 492 nm) was measured with a microplate reader. The reference wavelength was set to 620 nm.

[Example 1-2: Reference Absorbance Measurement and Calculation of Cell Proliferation Rate]

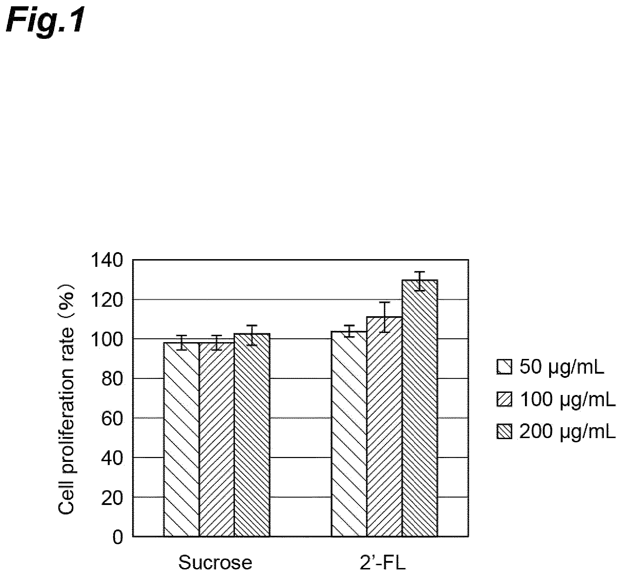

Human normal epidermal keratinocytes were cultured and the absorbance of formazan was measured in the same manner as in Example 1-1, except that no test sample was added. The obtained absorbance was designated as Ab, and the cell proliferation rate represented by the following formula (1) was determined. The results are shown in Fig. 1.

$$\text{Cell proliferation rate (\%)} = 100 \times As/Ab \ ... \ (1)$$

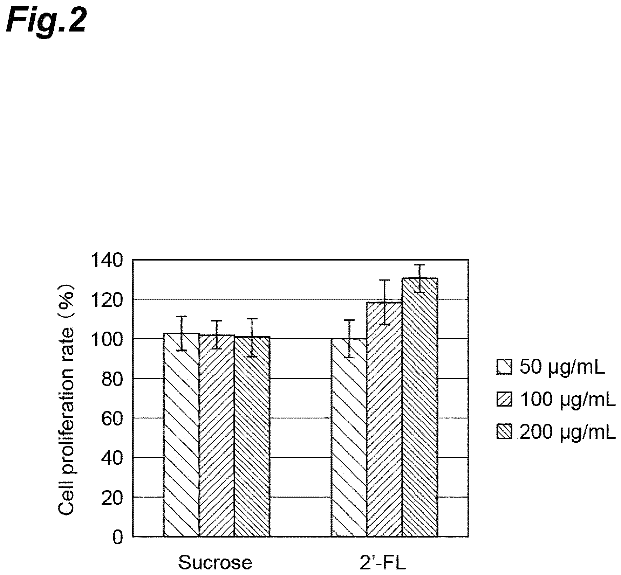

As shown in Fig. 1, 2'-FL improved the proliferation activity of normal epidermal cells. At least in the range where the content of 2'-FL in the medium was 50 μg/mL to 200 μg/mL, the epidermal cell proliferation activity increased as the content of 2'-FL increased.

[Example 2]

The fibroblast proliferation activity of 2'-fucosyllactose was evaluated.

[Example 2-1: Absorbance Measurement]

Human normal dermal fibroblasts (NB1RGB) with a PDL of 30 or less and human senescent dermal fibroblasts (NB1RGB) with a PDL of 50 or more were each cultured using DMEM containing 10% FBS. The cultured cells were collected by trypsin treatment. The collected cells were seeded in a 96-well microplate at a concentration of 5.0 x $10^3$ cells/well and cultured overnight. Thereafter, the cells were cultured for 2 days at 37°C in a medium for fibroblast proliferation (FBS-free) to which a test sample (sucrose or 2'-fucosyllactose) was added to a concentration of 50 μg/mL, 100 μg/mL, or 200 μg/mL. The medium was replaced with FBS-free DMEM medium, then 10 μL/well of 5 mg/mL 3-[4,5-dimethylthiazol-2-yl]-2,5-diphenyltetrazolium bromide (MTT) was added, and the cells were cultured at 37°C for 3 hours. After completion of culture, formazan produced in the cells was extracted with 100 μL of dimethyl sulfoxide (DMSO). After extraction, the absorbance of formazan (As, 492 nm) was measured with a microplate reader. The reference wavelength was set to 620 nm.

[Example 2-2: Reference Absorbance Measurement and Calculation of Cell Proliferation Rate

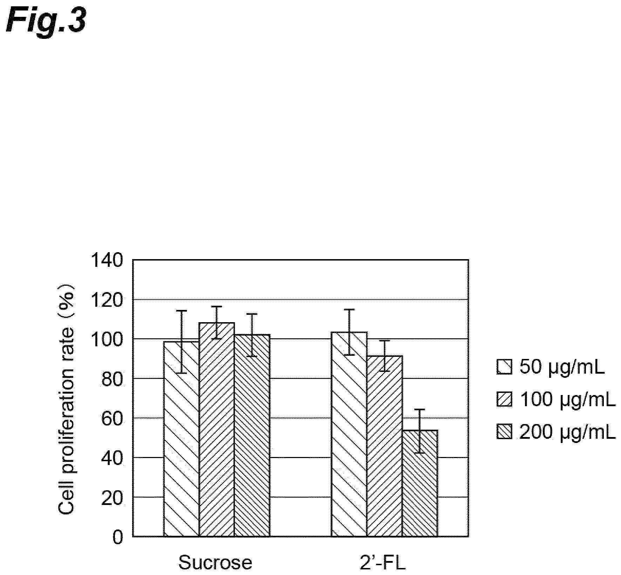

Human normal dermal fibroblasts with a PDL of 30 or less and human senescent fibroblasts with a PDL of 50 or more were each cultured and the absorbance of formazan was measured in the same manner as in Example 2-1, except that no test sample was added. The obtained absorbance was designated as Ab, and the cell proliferation rate represented by the above formula (1) was determined for each case of PDL of 30 or less and PDL of 50 or more. The results for PDL of 30 or less are shown in Fig. 2. The results for PDL of 50 or more are shown in Fig. 3.
As shown in Fig. 2, 2'-FL improved the proliferation activity of normal fibroblasts (PDL of 30 or less). At least in the range where the content of 2'-FL in the medium was 50 μg/mL to 200 μg/mL, the proliferation activity of normal fibroblasts increased as the content of 2'-FL increased.
As shown in Fig. 3, 2'-FL suppressed the proliferation of senescent fibroblasts (PDL of 50 or more). At least in the range where the content of 2'-FL in the medium was 50 μg/mL to 200 μg/mL, the proliferation-suppressing activity on senescent fibroblasts increased as the content of 2'-FL increased.

[Example 3]

The effect of 2'-fucosyllactose on the longevity-related gene (GLS1) was evaluated.

[Example 3-1: Cell Treatment]

Human normal dermal fibroblasts (NB1RGB, PDL of 30 or less) were cultured using DMEM containing 10% FBS. The cultured cells were collected by trypsin treatment. The collected cell-containing liquid was diluted with DMEM containing 10% FBS to a cell concentration of 5.0 x $10^5$ cells, seeded in a 10 cm dish, and cultured until 60% confluent. Thereafter, the cultured cells were subjected to a treatment of exposure to a medium containing 50 $\mu$M hydrogen peroxide (FBS-free) for 2 hours once a day for 3 days. Then, the medium was replaced with DMEM containing 5% FBS, and after 2 days, the medium (medium A) was collected. Dermal fibroblasts not exposed to hydrogen peroxide were diluted with DMEM containing 10% FBS to a concentration of 5.0 x $10^5$ cells, seeded in a 10 cm dish, and cultured for 24 hours. Thereafter, half the total volume of medium A used for culturing the dermal fibroblasts after hydrogen peroxide treatment was added, and the cells were cultured until 90% confluent.

[Example 3-2: Sample Preparation for GLS1 Expression Analysis]

The dermal fibroblasts prepared in [Example 3-1] were diluted with DMEM medium containing 10% FBS to a concentration of 8.0 x $10^4$ cells, seeded in a 6-well plate, and cultured until 80% confluent. Next, the medium was replaced with a medium for fibroblast proliferation (containing neither DMEM nor FBS) to which a test sample (lactose or 2'-fucosyllactose) was added to a concentration of 50 $\mu$g/mL or 200 $\mu$g/mL, and after culturing for 24 hours, the culture supernatant was removed. After washing the cells twice with cold PBS, proteins were extracted using RIPA buffer (50 mmol/L Tris-HCl buffer (pH 7.9), 150 mmol/L NaCl, 1% (w/v) Nonidet P40 substitute, 0.5% (w/v) sodium deoxycholate, 0.1% (w/v) SDS). The extracted proteins were quantified by the Bradford assay.

[Example 3-3: Reference Cell Treatment]

Except that no test sample was added, skin fibroblasts prepared in [Example 3-1] and skin fibroblasts cultured using DMEM containing 10% FBS were cultured in the same manner as in [Example 3-2], and proteins were extracted. The extracted proteins were quantified by the Bradford assay.

[Example 3-4: GLS1 Expression Analysis]

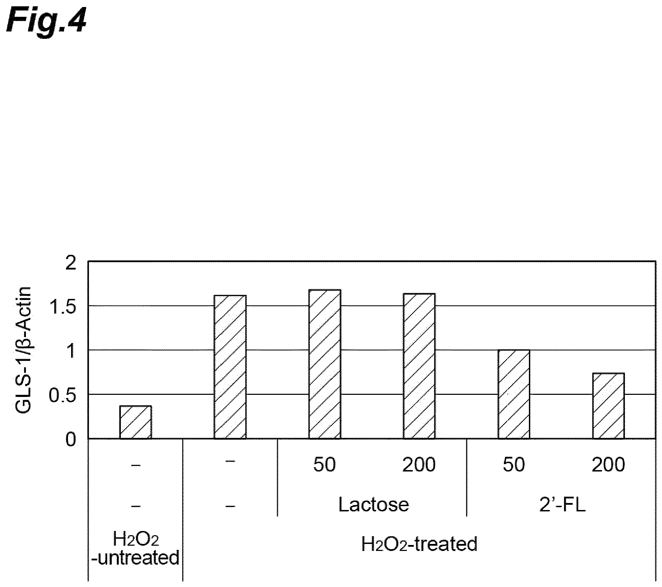

Western blot analysis was performed using antibodies against GLS1 and $\beta$-actin to measure protein levels at 24 hours. Equal amounts of protein were added to a 10% acrylamide gel. An antibody against GLS1 (manufactured by Cell Signaling Technology) and an antibody against $\beta$-actin (HRP-labeled) (manufactured by Proteintech) were used according to the manufacturer's instructions. For GLS1 only, HRP (manufactured by Proteintech) was used as the secondary antibody, and color was developed with 3,3'-diaminobenzidine (DAB). The intensity of the color development was quantified using the open-source software "ImageJ" and is shown in Fig. 4.
As shown in Fig. 4, 2'-FL suppressed the expression of GLS1. At least in the range where the content of 2'-FL in the medium was 50 $\mu$g/mL to 200 $\mu$g/mL, the GLS1 expression-suppressing effect increased as the content of 2'-FL increased.

[Example 4]

The viability of tunicamycin-treated epidermal keratinocytes was evaluated.

[Example 4-1: Absorbance Measurement]

Human normal epidermal keratinocytes (HaCaT) were cultured using DMEM containing 10% FBS. The cultured cells were collected by trypsin treatment. The collected cells were seeded in a 96-well plate, and when they reached 100% confluence, tunicamycin, an agent that induces endoplasmic reticulum stress, was added at 0.1 $\mu$g/mL, and the cells were cultured for 24 hours. A group was also prepared in which FBS-free DMEM medium containing a test sample (2'-fucosyllactose) at 50 $\mu$g/mL, 100 $\mu$g/mL, or 200 $\mu$g/mL was added simultaneously with tunicamycin, and cultured for 24 hours. After completion of culture, the viability of human normal epidermal keratinocytes was evaluated using Cell Counting Kit-8 (manufactured by Dojindo Laboratories). The measurement wavelength (As) was set to 450 nm, and the reference wavelength was set to 620 nm.

[Example 4-2: Reference Absorbance Measurement and Calculation of Cell Viability]

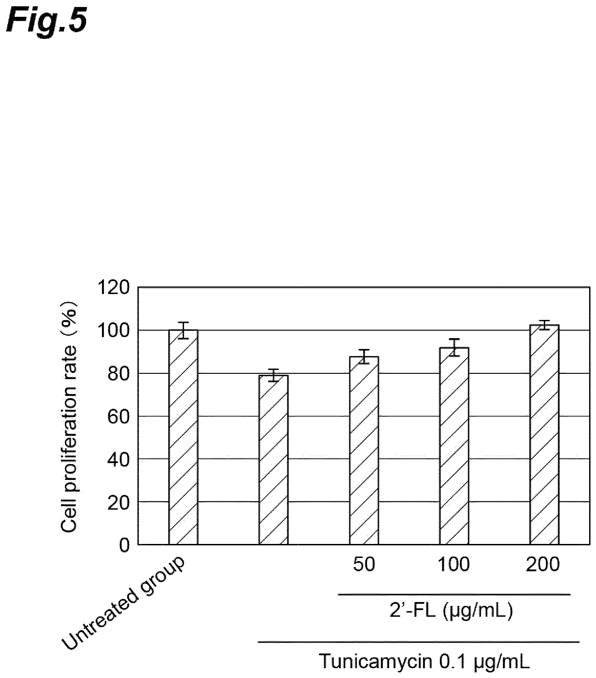

Human normal epidermal keratinocytes were cultured and the absorbance at a measurement wavelength of 450 nm was measured in the same manner as in Example 4-1, except that tunicamycin and the test sample were not added. The obtained absorbance was designated as Ab, and the cell viability represented by the above formula (1) was determined. The results are shown in Fig. 5.

As shown in Fig. 5, 2'-FL ameliorated endoplasmic reticulum stress in human normal epidermal keratinocytes. At least in the range where the content of 2'-FL in the medium was 50 $\mu$g/mL to 200 $\mu$g/mL, the ameliorating effect on endoplasmic reticulum stress in human normal epidermal keratinocytes increased as the content of 2'-FL increased.

[Example 5]

The viability of tunicamycin-treated dermal fibroblasts was evaluated.

[Example 5-1: Absorbance Measurement]

Human normal dermal fibroblasts (NB1RGB) with a PDL of 30 or less were cultured using DMEM containing 10% FBS. The cultured cells were collected by trypsin treatment. The collected cells were seeded in a 96-well plate, and when they reached 100% confluence, tunicamycin was added at 0.3 $\mu$g/mL, and the cells were cultured for 24 hours. A group was also prepared in which FBS-free DMEM medium containing a test sample (2'-fucosyllactose) at 50 $\mu$g/mL, 100 $\mu$g/mL, or 200 $\mu$g/mL was added simultaneously with tunicamycin, and cultured for 24 hours. After completion of culture, the viability of human normal dermal fibroblasts was evaluated using Cell Counting Kit-8 (manufactured by Dojindo Laboratories). The measurement wavelength (As) was set to 450 nm, and the reference wavelength was set to 620 nm.

[Example 5-2: Reference Absorbance Measurement and Calculation of Cell Viability]

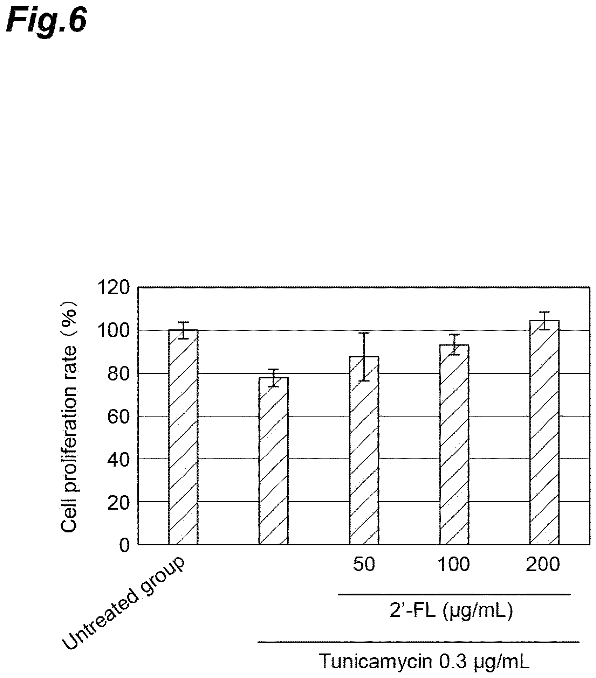

Human normal dermal fibroblasts with a PDL of 30 or less were cultured and the absorbance at a measurement wavelength of 450 nm was measured in the same manner as in Example 5-1, except that tunicamycin and the test sample were not added. The obtained absorbance was designated as Ab, and the cell viability represented by the above formula (1) was determined. The results are shown in Fig. 6.

As shown in Fig. 6, 2'-FL ameliorated endoplasmic reticulum stress in human normal dermal fibroblasts with a PDL of 30 or less. At least in the range where the content of 2'-FL in the medium was 50 $\mu$g/mL to 200 $\mu$g/mL, the ameliorating effect on endoplasmic reticulum stress in human normal dermal fibroblasts with a PDL of 30 or less increased as the content of 2'-FL increased.

[Example 6]

The laminin secretion amount of tunicamycin-treated epidermal keratinocytes was evaluated.

[Example 6-1: Sample Preparation for Laminin Analysis]

Human normal epidermal keratinocytes (HaCaT) were cultured using DMEM containing 10% FBS. The cultured cells were collected by trypsin treatment. The collected cells were seeded in a 24-well plate, and when they reached 100% confluence, tunicamycin was added at 0.1 $\mu$g/mL, and after 24 hours, the culture supernatant was collected. A group was also prepared in which FBS-free DMEM medium containing a test sample (2'-fucosyllactose) at 50 $\mu$g/mL, 100 $\mu$g/mL, or 200 $\mu$g/mL was added simultaneously with tunicamycin, and after 24 hours, the culture supernatant was collected. The culture supernatant was centrifuged, and the resulting supernatant was used as a sample. Further, the cells after removing the culture supernatant were washed twice with PBS, and 0.20 mL of RIPA buffer was added to extract total cell proteins. This total cell protein concentration was measured by the Bradford assay.

[Example 6-2: Reference Cell Treatment]

Human normal epidermal keratinocytes were cultured and the culture supernatant was collected in the same manner as in Example 6-1, except that tunicamycin and the test sample were not added. The culture supernatant was centrifuged, and the resulting supernatant was used as a sample. Further, the cells after removing the culture supernatant were washed twice with PBS, and 0.20 mL of RIPA buffer was added to extract total cell proteins. This total cell protein concentration was measured by the Bradford assay.

[Example 6-3: Laminin Expression Analysis]

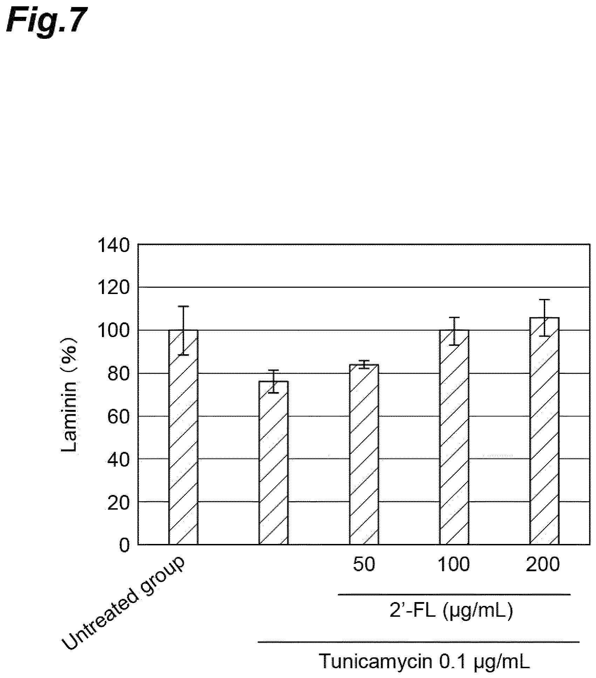

An Enzyme-Linked Immunosorbent Assay (ELISA) method was performed using an antibody against laminin to measure the amount of laminin in the sample. An anti-laminin antibody (in-house manufactured product, product number F-54, Clone No. HL-4H3) was used as described in the datasheet. HRP (manufactured by SeraCare Life Sciences) was used as the secondary antibody, and after color development with 3,3',5,5'-tetramethylbenzidine (TMB), the reaction was stopped with 1N sulfuric acid. After stopping the reaction, the absorbance at a measurement wavelength of 450 nm was measured with a microplate reader. The reference wavelength was set to 620 nm. The laminin secretion amount was defined as the amount of laminin measured by ELISA divided by the total cell protein amount and standardized. The results are shown in Fig. 7.

As shown in Fig. 7, 2'-FL restored laminin secretion in human normal epidermal keratinocytes. At least in the range where the content of 2'-FL in the medium was 50 $\mu$g/mL to 200 $\mu$g/mL, the recovery effect on laminin secretion in human normal epidermal keratinocytes increased as the content of 2'-FL increased.

[Example 7]

The laminin secretion amount of tunicamycin-treated dermal fibroblasts was evaluated.

[Example 7-1: Sample Preparation for Laminin Analysis]

Human normal dermal fibroblasts (NB1RGB) with a PDL of 30 or less were cultured using DMEM containing 10% FBS. The cultured cells were collected by trypsin treatment. The collected cells were seeded in a 24-well plate, and when they reached 100% confluence, tunicamycin at 0.3 $\mu$g/mL was added, and the culture supernatant was collected after 24 hours. A group was also prepared in which FBS-free DMEM medium containing the test sample (2'-fucosyllactose) at 50 $\mu$g/mL, 100 $\mu$g/mL, or 200 $\mu$g/mL was added simultaneously with tunicamycin, and the culture supernatant was collected after 24 hours. The culture supernatant was centrifuged, and the resulting supernatant was used as a sample. Furthermore, the cells after removing the culture supernatant were washed twice with PBS, and 0.20 mL of RIPA buffer was added to extract total cell protein. This total cell protein concentration was measured by the Bradford assay.

[Example 7-2: Reference Cell Treatment]

Human normal dermal fibroblasts with a PDL of 30 or less were cultured in the same manner as in Example 7-1, except that tunicamycin and the test sample were not added, and the culture supernatant was collected. The culture supernatant was centrifuged, and the resulting supernatant was used as a sample. Furthermore, the cells after removing the culture supernatant were washed twice with PBS, and 0.20 mL of RIPA buffer was added to extract total cell protein. This total cell protein concentration was measured by the Bradford assay.

[Example 7-3: Laminin Expression Analysis]

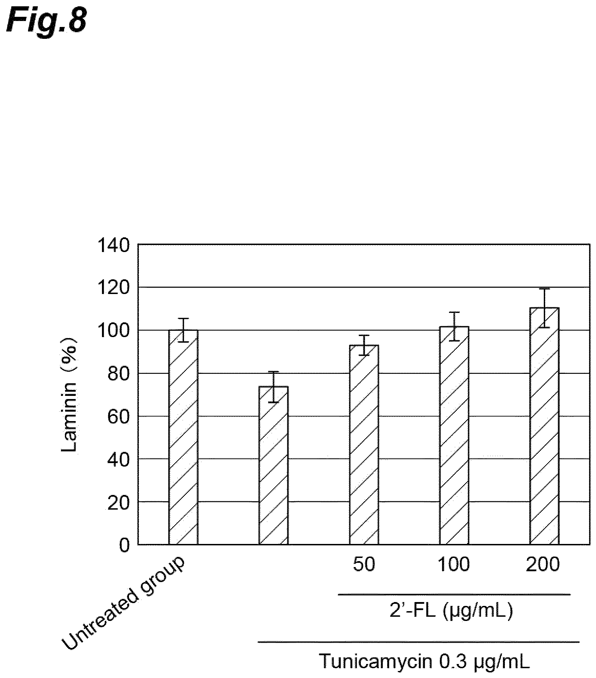

An Enzyme-Linked Immunosorbent Assay (ELISA) method was performed using an antibody against laminin to measure the amount of laminin in the samples. The anti-laminin antibody (in-house manufactured product, product number F-54, Clone No. HL-4H3) was used as described in the datasheet. HRP (manufactured by SeraCare Life Sciences) was used as the secondary antibody, color was developed with 3,3',5,5'-tetramethylbenzidine (TMB), and then the reaction was stopped with 1N sulfuric acid. After stopping the reaction, the absorbance at a measurement wavelength of 450 nm was measured using a microplate reader. The reference wavelength was set to 620 nm. The laminin amount measured by ELISA was normalized by dividing by the total cell protein amount, and this was taken as the laminin secretion amount. The results are shown in Fig. 8.

As shown in Fig. 8, 2'-FL restored laminin secretion in human normal dermal fibroblasts with a PDL of 30 or less. At least in the range where the content of 2'-FL in the medium was 50 $\mu$g/mL to 200 $\mu$g/mL, the recovery effect on laminin secretion in human normal dermal fibroblasts with a PDL of 30 or less increased as the content of 2'-FL increased.

[Example 8]

The effect of 2'-FL on the type I collagen expression level was evaluated.

[Example 8-1: Sample Preparation for Type I Collagen Expression Analysis]

Human normal dermal fibroblasts (NB1RGB) with a PDL of 30 or less were cultured in DMEM (Dulbecco's Modified Eagle Medium, manufactured by Nissui Pharmaceutical Co., Ltd.) containing 10% FBS. The cultured cells were collected by trypsin treatment. The collected cells were seeded in a 96-well plate, and when they reached 100% confluence, FBS-free DMEM containing the test sample (2'-FL and sucrose) at 50 µg/mL, 100 µg/mL, or 200 µg/mL was added, and after culturing for 48 hours in a 37°C, 5% $CO_2$ incubator, the culture supernatant was collected. The culture supernatant was centrifuged, and the resulting supernatant was used as a sample.

[Example 8-2: Preparation of Control Sample]

The culture supernatant was collected and used as a sample in the same manner as in [Example 8-1], except that FBS-free DMEM not containing the test sample was used instead of FBS-free DMEM containing the test sample.

[Example 8-3: Type I Collagen Expression Analysis]

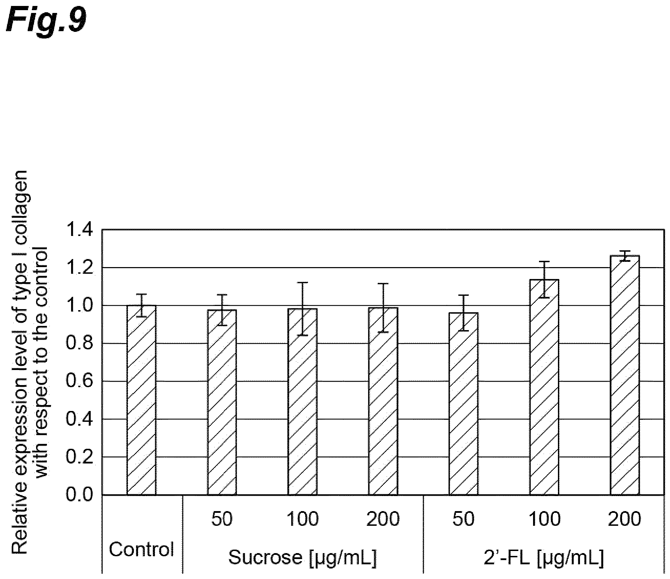

An Enzyme-Linked Immunosorbent Assay (ELISA) method was performed using an antibody against type I collagen to measure the amount of type I collagen in the samples. Two types of anti-type I collagen antibodies (in-house manufactured product [product number F-56, clone number I-8H5] and one manufactured by Proteintech) were used as described in their datasheets. An HRP-labeled antibody (manufactured by SeraCare Life Sciences) was used as the secondary antibody, color was developed with 3,3',5,5'-tetramethylbenzidine (TMB), and then the reaction was stopped with 1N sulfuric acid. After stopping the reaction, the absorbance at a measurement wavelength of 450 nm was measured using a microplate reader. The reference wavelength was set to 620 nm. The relative value of the type I collagen amount when each test sample was added was calculated, with the type I collagen amount in the control set to 1. Furthermore, the measurement of type I collagen was performed three times, and Fig. 9 shows the relative expression level of type I collagen with respect to the control as mean ± standard deviation.

As shown in Fig. 9, 2'-FL at a concentration of at least 100 µg/mL or more increased the expression level of type I collagen in human normal epidermal keratinocytes. At least in the range where the content of 2'-FL in the medium was 100 µg/mL to 200 µg/mL, the effect of increasing the type I collagen expression level increased as the content of 2'-FL increased.

[Example 9]

The effect of 2'-FL on the IL-1α expression level was evaluated.

[Example 9-1: Sample Preparation for IL-1α Expression Analysis]

Human normal epidermal keratinocytes (HaCaT) were cultured in DMEM containing 10% FBS. The cultured cells were collected by trypsin treatment. The collected cells were seeded in a 48-well plate at a cell density of 6 x $10^4$ cells/well and cultured for 24 hours in a 37°C, 5% $CO_2$ incubator. After culturing, the cells were precultured for 2 hours in FBS-free DMEM containing the test sample (2'-FL and sucrose) at 50 µg/mL, 100 µg/mL, or 200 µg/mL, or in FBS-free DMEM not containing the test sample. Thereafter, the medium was replaced with FBS-free DMEM containing the test sample at 50 µg/mL, 100 µg/mL, or 200 µg/mL and 20 µM $H_2O_2$, or with FBS-free DMEM not containing the test sample but containing 20 µM $H_2O_2$, respectively, and after culturing for an additional 24 hours in a 37°C, 5% $CO_2$ incubator, the culture supernatant was collected. The culture supernatant was centrifuged, and the resulting supernatant was used as a sample.

[Example 9-2: Preparation of Control Sample]

The culture supernatant was collected and used as a sample in the same manner as in [Example 9-1], except that FBS-free DMEM not containing $H_2O_2$ and the test sample was used instead of FBS-free DMEM containing $H_2O_2$ and the test sample.

[Example 9-3: IL-1α Expression Analysis]

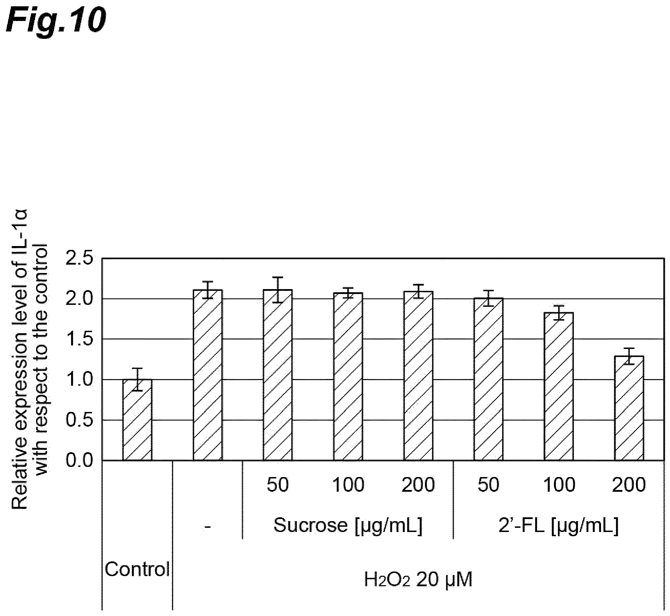

Since most IL-1α is released extracellularly, the IL-1α expression level was evaluated by measuring IL-1α in the supernatant medium by ELISA (AuthentiKine™ IL-1 alpha ELISA Kit, manufactured by Proteintech). The measurement wavelength was set to 450 nm, and the reference wavelength was set to 630 nm. The relative value of the IL-1α amount when each test sample was added was calculated, with the IL-1α amount in the control set to 1. Furthermore, the

measurement of IL-1$\alpha$ was performed three times, and Fig. 10 shows the relative expression level of IL-1$\alpha$ with respect to the control as mean $\pm$ standard deviation.

As shown in Fig. 10, 2'-FL suppressed the $H_2O_2$-induced promotion of IL-1 $\alpha$ expression in human normal epidermal keratinocytes. At least in the range where the content of 2'-FL in the medium was 50 $\mu$g/mL to 200 $\mu$g/mL, the suppressive effect on IL-1$\alpha$ expression increased as the content of 2'-FL increased.

[Example 10]

The effect of 2'-FL on a longevity-related gene (GLS1) was evaluated.

[Example 10-1: Sample Preparation for GLS1 Expression Analysis]

A. Human embryonic kidney cells (HEK293)

Human embryonic kidney cells (HEK293) were cultured in DMEM containing 10% FBS. The cultured cells were collected by trypsin treatment. The collected cells were seeded in a 6-well plate, and when they reached 100% confluence, DMEM containing 2'-FL as a test sample at 50 $\mu$g/mL, 200 $\mu$g/mL, or 1000 $\mu$g/mL, and lactose at 50 $\mu$g/mL or 200 $\mu$g/mL was added, and cultured for 24 hours in a 37°C, 5% $CO_2$ incubator. The culture supernatant was removed, the cells were washed twice with phosphate-buffered saline (PBS), and 0.20 mL of RIPA buffer (manufactured by Nacalai Tesque, Inc.) was added to extract total protein. Protein in each protein extract was quantified by the Bradford assay.

B. Human gastric undifferentiated carcinoma cells (KKLS)

Human gastric undifferentiated carcinoma cells (KKLS) were cultured in RPMI 1640 (manufactured by Nissui Pharmaceutical Co., Ltd.) containing 10% FBS. The cultured cells were collected by trypsin treatment. The collected cells were seeded in a 6-well plate, and when they reached 100% confluence, RPMI 1640 containing the test sample (2'-FL and lactose) at 50 $\mu$g/mL, 200 $\mu$g/mL, or 1000 $\mu$g/mL was added, and cultured for 24 hours in a 37°C, 5% $CO_2$ incubator. The culture supernatant was removed, the cells were washed twice with PBS, and 0.20 mL of RIPA buffer was added to extract total protein. Protein in each protein extract was quantified by the Bradford assay.

C. Mouse microglial cells (BV2)

Mouse microglial cells (BV2) were cultured in glutamine-containing DMEM. The cultured cells were collected by trypsin treatment. The collected cells were seeded in a 6-well plate at a cell density of 4 x 10$^5$ cells/well and cultured for 24 hours in a 37°C, 5% $CO_2$ incubator. After culturing, glutamine-free DMEM containing the test sample (2'-FL and lactose) at 50 $\mu$g/mL, 200 $\mu$g/mL, or 1000 $\mu$g/mL was added, and cultured for 24 hours in a 37°C, 5% $CO_2$ incubator. The culture supernatant was removed, the cells were washed twice with PBS, and 0.20 mL of RIPA buffer was added to extract total protein. Protein in each protein extract was quantified by the Bradford assay.

D. Human gingival fibroblasts (Gin-1)

Human gingival fibroblasts (Gin-1) were cultured in DMEM containing 10% FBS. The cultured cells were collected by trypsin treatment. Cells in a state where GLS1 was expressed without addition of the test sample were used for the test. Cells collected by trypsin treatment were seeded in a 6-well plate, and when they reached 80% confluence, DMEM containing the test sample (2'-FL and lactose) at 50 $\mu$g/mL, 200 $\mu$g/mL, or 1000 $\mu$g/mL was added, and cultured for 24 hours in a 37°C, 5% $CO_2$ incubator. The culture supernatant was removed, the cells were washed twice with PBS, and 0.20 mL of RIPA buffer was added to extract total protein. Protein in each protein extract was quantified by the Bradford assay.

E. Human umbilical vein endothelial cells (HUVEC)

Human umbilical vein endothelial cells (HUVEC) were cultured in DMEM + 10% FBS + 5 mM glucose (normal medium). The cultured cells were collected by trypsin treatment. The collected cells were seeded in a 10 cm dish and cultured for 24 hours in a 37°C, 5% $CO_2$ incubator. Thereafter, a treatment of exposing the cells to DMEM + 10% FBS + 30 mM glucose (high glucose medium) for 10 hours and to normal medium for 14 hours was performed for 7 days. After 7 days, normal medium containing the test sample (2'-FL and lactose) at 50 $\mu$g/mL, 200 $\mu$g/mL, or 1000 $\mu$g/mL was added, and cultured for 24 hours in a 37°C, 5% $CO_2$ incubator. The culture supernatant was removed, the cells were washed twice with PBS, and 0.20 mL of RIPA buffer was added to extract total protein. Protein in each protein extract was quantified by the Bradford assay.

[Example 10-2: Preparation of Control Samples]

A. Human embryonic kidney cells (HEK293)

Protein extracts were collected in the same manner as in [Example 10-1 A. Human embryonic kidney cells (HEK293)], except that DMEM not containing the test sample was used instead of DMEM containing the test sample, and protein in each protein extract was quantified by the Bradford assay.

B to E. Human gastric undifferentiated carcinoma cells (KKLS),

Mouse microglial cells (BV2), Human gingival fibroblasts (Gin-1), Human umbilical vein endothelial cells (HUVEC)

Protein extracts were collected in the same manner as in [Example 10-1 B. Human gastric undifferentiated carcinoma cells (KKLS)], [Example 10-1 C. Mouse microglial cells (BV2)], [Example 10-1 D. Human gingival fibroblasts (Gin-1)], and [Example 10-1 E. Human umbilical vein endothelial cells (HUVEC)], respectively, except that a medium same as the above-mentioned medium but not containing the test sample was used instead of the medium containing the test sample, and protein in each protein extract was quantified by the Bradford assay.

[Example 10-3. GLS1 Expression Analysis]

A. Human embryonic kidney cells (HEK293)

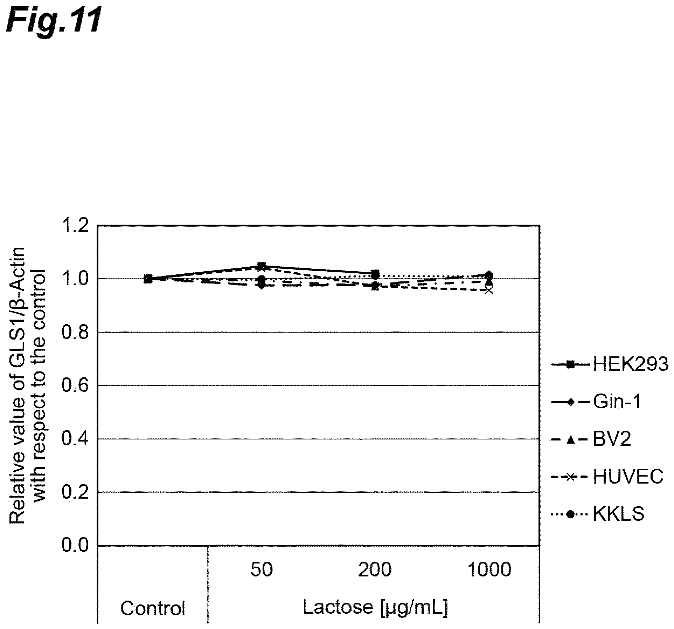

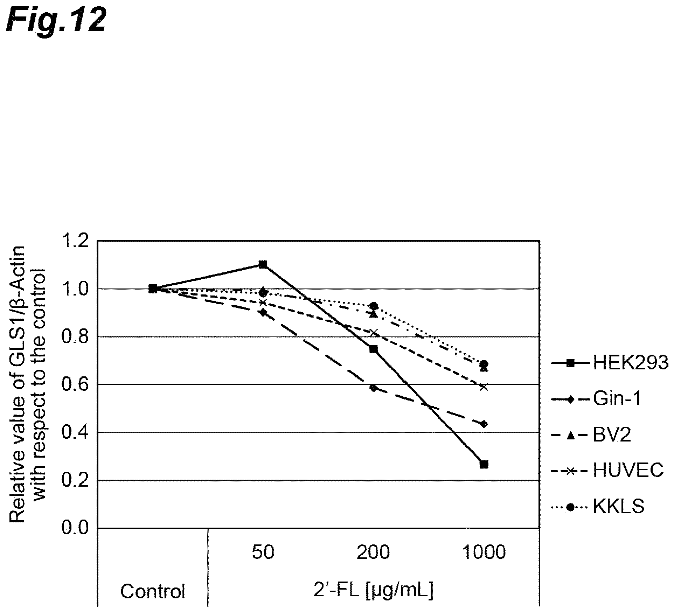

Western blot analysis was performed on each protein extract using an anti-GLS1 antibody and an anti-$\beta$-actin antibody. Specifically, each protein extract was added to a 10% acrylamide gel such that the protein amounts were the same. The anti-GLS1 antibody (manufactured by Cell Signaling) and HRP-labeled anti-$\beta$-actin antibody (manufactured by Proteintech) were used according to the manufacturer's instructions. For GLS1, an HRP-labeled antibody (manufactured by Proteintech) was used as the secondary antibody. HRP was developed with 3,3'-diaminobenzidine (DBA). The intensity of color development was quantified using the open-source software 'ImageJ'. The relative value of the GLS1 amount relative to the $\beta$-actin amount when each test sample was added was calculated, with the GLS1 amount relative to the $\beta$-actin amount in the control set to 1. The said relative values when lactose was used are shown in Fig. 11, and the said relative values when 2'-FL was used are shown in Fig. 12.

B to E. Human gastric undifferentiated carcinoma cells (KKLS),

Mouse microglial cells (BV2), Human gingival fibroblasts (Gin-1), Human umbilical vein endothelial cells (HUVEC)

An ELISA method was performed using an antibody against GLS1 to measure the amount of GLS1 in the samples. Two types of anti-GLS1 antibodies (manufactured by Cell Signaling and Proteintech) were used as described in their datasheets. An HRP-labeled antibody (manufactured by SeraCare Life Sciences) was used as the secondary antibody, color was developed with 3,3',5,5'-tetramethylbenzidine (TMB), and then the reaction was stopped with 1N sulfuric acid. After stopping the reaction, the absorbance at a measurement wavelength of 450 nm was measured using a microplate reader. The reference wavelength was set to 620 nm. The relative value of the GLS1 amount relative to the $\beta$-actin amount when each test sample was added was calculated, with the GLS1 amount relative to the $\beta$-actin amount in the control set to 1. The said relative values when lactose was used are shown in Fig. 11, and the said relative values when 2'-FL was used are shown in Fig. 12.
As shown in Figs. 11 and 12, 2'-FL suppressed GLS1 expression in human umbilical vein endothelial cells and human gingival fibroblasts, and 2'-FL at a concentration of at least 200 $\mu$g/mL or more suppressed GLS1 expression in human embryonic kidney cells, human gastric undifferentiated carcinoma cells, and mouse microglial cells. Furthermore, at least in the range where the content of 2'-FL in the medium was 50 $\mu$g/mL to 1000 $\mu$g/mL, the suppressive effect on GLS1 expression in human umbilical vein endothelial cells and human gingival fibroblasts increased as the content of 2'-FL increased. At least in the range where the content of 2'-FL in the medium was 200 to 1000 $\mu$g/mL, the suppressive effect on GLS1 expression in human embryonic kidney cells, human gastric undifferentiated carcinoma cells, and mouse microglial cells increased as the content of 2'-FL increased.

**Claims**

1. A prophylactic or ameliorating agent for cell aging, comprising 2'-fucosyllactose.

2. The prophylactic or ameliorating agent according to claim 1, for promoting proliferation of normal cells.

3. The prophylactic or ameliorating agent according to claim 1, for removing senescent cells.

4. The prophylactic or ameliorating agent according to claim 1, wherein the cell aging is cell aging caused by GLS1 expression.

5. The prophylactic or ameliorating agent according to claim 1, wherein the cell aging is cell aging caused by endoplasmic reticulum stress.

6. The prophylactic or ameliorating agent according to claim 1, wherein the cell is a skin cell.

7. The prophylactic or ameliorating agent according to claim 1, wherein the cell is an epidermal keratinocyte.

8. The prophylactic or ameliorating agent according to claim 1, wherein the cell is a dermal fibroblast.

9. The prophylactic or ameliorating agent according to claim 1, which is at least one selected from the group consisting of an agent for treating skin wounds, an agent for promoting skin metabolism, a prophylactic or ameliorating agent for blemishes, a prophylactic or ameliorating agent for wrinkles, a prophylactic or ameliorating agent for sagging, and a prophylactic or ameliorating agent for rough skin.

10. The prophylactic or ameliorating agent according to claim 1, wherein the cell is a kidney cell, a stomach cell, a microglial cell, a gingival cell, or a vascular endothelial cell.

11. The prophylactic or ameliorating agent according to claim 1, which is at least one selected from the group consisting of a prophylactic or ameliorating agent for glomerular sclerosis, prophylactic or ameliorating agent for decline in renal function, a prophylactic or ameliorating agent for decrease in gastric acid secretion from stomach parietal cells, a prophylactic or ameliorating agent for decrease in pepsinogen secretion from stomach chief cells, a prophylactic or ameliorating agent for cognitive decline, a prophylactic or ameliorating agent for inflammation in gingiva, a prophylactic or ameliorating agent for arteriosclerosis, and a prophylactic or ameliorating agent for obesity, diabetes, or impaired glucose tolerance.

12. The prophylactic or ameliorating agent according to claim 1, which is a topical agent.

13. The prophylactic or ameliorating agent according to any one of claims 1 to 12, which is a cosmetic.

14. The prophylactic or ameliorating agent according to any one of claims 1 to 11, which is a food product.

**Fig.1**

# *Fig.2*

# Fig.3

# Fig.4

*Fig.5*

# Fig.6

# Fig.7

## Fig.8

EP 4 670 722 A1

Fig.9

26

# Fig.10

# Fig.11

# Fig.12

# EP 4 670 722 A1

## INTERNATIONAL SEARCH REPORT

| | International application No. |
|---|---|
| | **PCT/JP2024/006093** |

### A. CLASSIFICATION OF SUBJECT MATTER

*A61K 31/702*(2006.01)i; *A61K 8/60*(2006.01)i; *A61P 17/00*(2006.01)i; *A61P 17/02*(2006.01)i; *A61P 43/00*(2006.01)i; *A61Q 1/00*(2006.01)i; *A61Q 19/00*(2006.01)i; *A61Q 19/08*(2006.01)i
FI:  A61K31/702; A61K8/60; A61P17/00; A61P17/02; A61P43/00 107; A61Q1/00; A61Q19/00; A61Q19/08

According to International Patent Classification (IPC) or to both national classification and IPC

### B. FIELDS SEARCHED

Minimum documentation searched (classification system followed by classification symbols)

A61K31/702; A61K8/60; A61P17/00; A61P17/02; A61P43/00; A61Q1/00; A61Q19/00; A61Q19/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | KR 10-2018-0102431 A (COSMAX, INC.) 17 September 2018 (2018-09-17) claims 1, 5, 11-13, examples | 1–3, 5–14 |
| A | | 4 |
| X | WANG, J. et al. 2'-Fucosyllactose Ameliorates Oxidative Stress Damage in D-Galactose-Induced Aging Mice by Regulating Gut Microbiota and AMPK/SIRT 1/FOXO 1 Pathway. Foods, 2022, 11(2), 151 abstract, p. 10, line 14 to p. 13 line 2 | 1-3, 5-14 |
| A | | 4 |
| X | JP 2003-292444 A (KYOWA HAKKO KOGYO CO., LTD.) 15 October 2003 (2003-10-15) claims 5, 33, example 1, test examples 1, 2 | 1, 11, 14 |
| A | | 2-10, 12-13 |
| A | WO 2020/095971 A1 (UNIV TOKYO) 14 May 2020 (2020-05-14) | 1-14 |

☑ Further documents are listed in the continuation of Box C.      ☑ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **22 April 2024** | **14 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

| International application No. |
| --- |
| **PCT/JP2024/006093** |

### C.    DOCUMENTS CONSIDERED TO BE RELEVANT

| Category* | Citation of document, with indication, where appropriate, of the relevant passages | Relevant to claim No. |
| --- | --- | --- |
| A | JOHMURA, Y. et al. Senolysis by glutaminolysis inhibition ameliorates various age-associated disorders, Science, 2021, 371, 265-270 | 1-14 |

## INTERNATIONAL SEARCH REPORT
### Information on patent family members

International application No.

**PCT/JP2024/006093**

| Patent document cited in search report | | | Publication date (day/month/year) | Patent family member(s) | | | Publication date (day/month/year) |
|---|---|---|---|---|---|---|---|
| KR | 10-2018-0102431 | A | 17 September 2018 | (Family: none) | | | |
| JP | 2003-292444 | A | 15 October 2003 | US claims 4, 20, example 1, test example 1, 2 EP | 2003/0181401 1332759 | A1 A1 | |
| WO | 2020/095971 | A1 | 14 May 2020 | US EP CN | 2022/0119768 3878470 112996541 | A1 A1 A | |

Form PCT/ISA/210 (patent family annex) (July 2022)

32

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021065176 A
- KR 1020190067499
- WO 2012053849 A
- WO 2014138578 A
- WO 2000006115 A
- KR 1020110092602
- KR 1020160009738
- JP 2001206848 A
- JP H03049648 A
- JP H02207089 A

- WO 2016172183 A
- KR 1020180051688
- KR 1020190045079
- CN 109043542
- EP 3127543 A
- US 5002759 A
- US 5401723 A
- JP 2022112464 A
- JP 2020011930 A

**Non-patent literature cited in the description**

- **JOHMURA** ; **YOSHIKAZU et al.** Senolysis by glutaminolysis inhibition ameliorates various age-associated disorders. *Science*, 2021, vol. 371 (6526), 265-270
- **SHOHEI KASAI**. Removal of senescent glial cells: a key to preventing cognitive decline?. *Pharmacia*, 2019, vol. 55 (9), 889-889

- **MASAYOSHI SUDA** ; **IPPEI SHIMIZU** ; **TORU MINAMINO**. On vascular endothelial cell senescence. *Japanese Journal of Thrombosis and Hemostasis*, 2019, vol. 30 (3), 521-528